# EUROPEAN PATENT SPECIFICATION

(11) **EP 2 501 754 B1**
(45) Date of publication and mention of the grant of the patent: **08.08.2018**
(21) Application number: 10831000.4
(22) Date of filing: 18.11.2010
(51) Int. Cl.: A61K 35/14, A61K 31/722, C08K 11/00, C08K 3/00, C08K 3/16, C08K 5/521, C08L 5/08

(54) **NOVEL FORMULATION OF PHYSIOLOGICAL CHITOSAN-INORGANIC SALT SOLUTION/BLOOD MIXTURES FOR TISSUE REPAIR**
NEUE FORMULIERUNG AUS PHYSIOLOGISCHEM CHITOSAN, EINER ANORGANISCHEN SALZLÖSUNG UND BLUTMISCHUNGEN ZUR GEWEBEREPARATUR
NOUVELLE FORMULATION DE MÉLANGES SANG/SOLUTION SALÉE MINÉRALE-CHITOSANE PHYSIOLOGIQUE POUR RÉPARATION DE TISSU

(30) Priority: 19.11.2009 US 262786 P
(43) Date of publication of application: 26.09.2012
(73) Proprietor: Ortho Regenerative Technologies Inc., Kirkland, QC H9H 4R9 (CA)
(72) Inventor: OUYANG, Wei, Montréal, Québec H1H3X1 (CA); BUSCHMANN, Michael D, Vienna, Virginia 22181 (US); HOEMANN, Caroline, Vienna, Virginia 22181 (US); LAVERTU, Marc, Pointe-Claire, Québec H9R 3Z5 (CA); CHEVRIER, Anik, Pointe-Claire, Québec H9R 5V5 (CA)
(74) Representative: Griffin, Philippa Jane
(86) International application number: PCT/CA2010/001842
(87) International publication number: WO 2011/060544

(56) References cited:
- WO-A2-02/00272
- WO-A2-2006/015178
- CA-A1- 2 412 505
- CHANG S J ET AL: "Evaluation of chitosan/CaSO4/platelet-rich plasma microsphere composites as alveolus osteogenesis material", BIOMEDICAL ENGINEERING - APPLICATIONS, BASIS AND COMMUNICATIONS APRIL 2009 WORLD SCIENTIFIC PUBLISHING CO. PTE. LTD SGP, vol. 21, no. 2, April 2009 (2009-04), pages 115-122, XP009170511, DOI: 10.4015/S101623720900112X
- CHENG WEN-JUN ET AL: "Effect of injectable scaffolds material chitosan-beta-tricalcium phosphate with platelet-rich plasma on proliferation of bone marrow stromal cells in vitro", TISSUE ENGINEERING, vol. 12, no. 4, April 2006 (2006-04), page 1018, XP009170510, & 8TH ANNUAL MEETING OF THE TISSUE-ENGINEERING-SOCIETY-INTERNATIONAL (TESI); SHANGHAI, PEOPLES R CHINA; OCTOBER 22 -25, 2005 ISSN: 1076-3279
- SHEN E-CHIN ET AL: "Releasing growth factors from activated human platelets after chitosan stimulation: a possible bio-material for platelet-rich plasma preparation.", CLINICAL ORAL IMPLANTS RESEARCH OCT 2006, vol. 17, no. 5, October 2006 (2006-10), pages 572-578, XP002699281, ISSN: 0905-7161
- CHEVRIER ET AL: "Chitosan-glycerol phosphate/blood implants increase cell recruitment, transient vascularization and subchondral bone remodeling in drilled cartilage defects", OSTEOARTHRITIS AND CARTILAGE, BAILLIERE TINDALL, LONDON, GB, vol. 15, no. 3, 15 February 2007 (2007-02-15), pages 316-327, XP005887281, ISSN: 1063-4584, DOI: 10.1016/J.JOCA.2006.08.007
- MARCHAND C ET AL: "Solidification mechanisms of chitosan-glycerol phosphate/blood implant for articular cartilage repair", OSTEOARTHRITIS AND CARTILAGE, BAILLIERE TINDALL, LONDON, GB, vol. 17, no. 7, 1 July 2009 (2009-07-01), pages 953-960, XP026168856, ISSN: 1063-4584, DOI: 10.1016/J.JOCA.2008.12.002 [retrieved on 2009-06-11]
- CHENITE A ET AL: "Novel injectable neutral solutions of chitosan form biodegradable gels in situ", BIOMATERI, ELSEVIER SCIENCE PUBLISHERS BV., BARKING, GB, vol. 21, no. 21, 1 November 2000 (2000-11-01), pages 2155-2161, XP004216030, ISSN: 0142-9612, DOI: 10.1016/S0142-9612(00)00116-2

## Description

### TECHNICAL FIELD

The present description relates to a novel composition comprising blood/chitosan-inorganic salt mixtures, wherein physiological chitosan-inorganic salt solutions mixed with blood solidify faster than chitosan-β glycerol-phosphate solutions with chitosans of specific molecular weights.

### BACKGROUND ART

Chitosan is a linear polysaccharide composed of β-(1-4)-linked D-glucosamine (deacetylated unit) and N-acetyl-D-glucosamine (acetylated unit), which primarily results from the alkaline deacetylation of chitin. Chitosan can exist in many structural conformations, depending on a variety of factors that include the degree of hydration, the electrolyte environment and the complexity of original chitin mixture. Chitosan and its amino-substituted derivatives are bioerodible, biocompatible and biodegradable cationic polymers that have been advanced for a wide variety of applications, including tissue engineering, drug and gene delivery, pharmaceutical formulation, scaffolds for cell growth and cell encapsulation, wound healing and surface hemostasis.

A well known property of chitosan is its solubility at acidic pH (<6) and insolubility at neutral pH, making its use in solution with living cells and tissues problematic. Various publications (Chenite, international patent application publication No. WO 99/07416; Chenite et al., 2000, Biomater., 21: 2155-2161; Chenite et al., 2001, Carbohyd. Polym., 46: 39-47) describe that admixing a polyol-phosphate dibasic salt, i.e. glycerol-phosphate (GP), to an aqueous solution of chitosan can increase the pH of the solution while avoiding precipitation of the polymer. In the presence of these particular salts, chitosan solutions of substantial concentration (0.5-3%) and high molecular weight (> several hundred kDa) remain liquid, at low or room temperature, for a long period of time with physiologically acceptable neutral pH region between 6.8 and 7.2. These chitosan-glycerol phosphate solutions which can gel upon mild heating (for example from 4 to 37°C), are biocompatible, biodegradable and adhesive to human tissues, provide for new opportunities in the delivery of sensitive therapeutics.

Compositions containing blood and chitosan have been described previously (see for example U.S patent No. 7,148,209, the content of which is enclosed herewith by reference). Chitosan is known for being a thrombogenic polymer (e.g. it can accelerate the coagulation of blood). Chitosan-GP solutions were combined with sheep peripheral whole blood to form a thrombogenic mixture that solidified and adhered to a full-thickness cartilage defect by using a sheep repair model. The obtained results showed that solidification of a chitosan-glycerol phosphate/blood implant in microfracture defects improved cartilage repair compared with microfracture alone by increasing the amount of tissue and improving its biochemical composition and cellular organization (Hoemann et al., 2005, J. Bone Joint Surg., 87A: 2671-2686). A bilateral rabbit cartilage repair model was developed to study the effect of chitosan-GP/blood implants on cartilage repair following marrow stimulation. Results showed that chitosan-GP structurally stabilized the blood clots by inhibiting the clot retraction. Treatment of drilled defect with chitosan-GP/blood clots led to the formation of more integrated and hyaline repair tissue above a more porous and vascularised subchondral bone plate compared to drilling alone (Hoemann et al., 2007, Osteoarthritis & Cartilage, 15: 78-89).

Chitosan-GP/blood implants also increase cell recruitment, transient vascularisation, subchondral remodeling and modulate inflammatory and repair cell phenotype suggesting that these events are in part responsible for increase quantity and quality of repair tissue zone (Chevrier et al., 2007, Osteoarthritis & Cartilage, 15: 316-327; Hoemann et al., 2010, Am. J. Sports Med., 38, 9: 1845-56). Ultrastructure and compositional detail of chitosan-GP/blood clots, chitosan-GP alone and clots containing whole blood only were investigated by environment scanning electron microscopy (ESEM) in conjunction with energy dispersive X-ray analysis (EDS) (Iliescu et al., 2007, Microsc. Res. Tech., 71: 236-247). It was shown that chitosan formed a network structure in both chitosan-GP gel and chitosan-GP/blood clots. However this structure was altered by aldehyde fixation to produce artifactual aggregates of chitosan microparticles. EDS analysis showed that the majority of glycerol phosphate can diffuse freely from chitosan-GP gel. Solidification mechanisms of chitosan-glycerol phosphate/blood implants were investigated as well. Results showed that chitosan-GP/blood implants solidify through coagulation mechanisms involving thrombin generation, platelet activation and fibrin polymerization. Clotting factors can be used to shorten the *in situ* solidification time of chitosan-GP/blood implants in microdrilled cartilage defects (Marchand et al., 2009, Osteoarthritis & Cartilage, 17: 953-960). However, to facilitate their use in the clinic, the coagulation time of blood/chitosan composition should be shortened

There is still a need for an improved blood/chitosan implant that will solidify faster than known chitosan-GP/blood implants solutions. It would be highly desirable to be provided with fast-coagulating blood/chitosan implants for tissue repair.
WO 02/00272 describes a composition and method for the repair and regeneration of cartilage and other tissues.
WO 2006/015178 describes a growth factor encapsulation system for enhancing bone formation.
Chang et al., BioMedical Engineering, Vol. 21(2), April 2009, pages 15-122, describes the evaluation of chitosan/CaSO4/platelet-rich-plasma microsphere composites as alveolus osteogenesis material.
Chevrier et al., Osteoarthritis and Cartilage, Vol. 15(3), 15 February 2007, pages 316-327, reports that chitosan-glycerol phosphate/blood implants increase cell recruitment, transient vascularization, and subchondral bone remodelling in drilled cartilage defects.
Marchand et al., Osteoarthritis and Cartilage, Vol. 17(7), 1 July 2009, pages 953-960, describes solidification mechanisms of a chitosan-glycerol phosphate/blood implant for articular cartilage repair.

There is now provided a polymer composition comprising a blood component, a polymer and an inorganic acid. Such polymer composition is useful in repairing a tissue in a subject

According to a first aspect, the present application provides a polymer composition comprising a blood component, a polymer and at least one inorganic salt as well as a polymer composition consisting essentially of a blood component, a polymer and at least one inorganic salt. In an embodiment, the polymer composition further comprises or contains a mineral acid or an organic acid, such as, for example, hydrochloric acid, nitric acid, phosphoric acid, sulfuric acid, boric acid, hydrofluoric acid and/or hydrobromic acid. In an embodiment, the polymer is chitosan, chitin, hyaluronan, glycosaminoglycan, chondroitin sulfate, keratin sulfate, dermatan sulfate, heparin and/or heparin sulfate. In another embodiment, the inorganic salt is sodium salt, chloride salt, potassium salt, calcium salt, magnesium salt, phosphate salt, sulfate salt and/or carboxylate salt. In yet a further embodiment, the at least one inorganic salt is NaCl. KCl, CsCl, CaCl₂, CsF, KClO₄, NaNO₃ and/or CaSO₄. In still another embodiment, the blood component is whole blood, processed blood, venous blood, arterial blood, blood from bone, blood from bone-marrow, bone marrow, umbilical cord blood and/or placenta blood. In still a further embodiment, the composition is a gel. In another embodiment, the blood component is coagulated. In another embodiment, the polymer composition has a pH between 6.0 and 7.8, between 6.2 and 6.7 or of 6.6. In yet another embodiment, the polymer composition has an osmolality between 200 mOsm/kg and 600 mOsm/kg, between 326 mOsm/kg and 356 mOsm/kg, or of 354 mOsm/kg. In still another embodiment, the polymer is chitosan with a degree of deacetylation (DDA) between 20% to 100% or between 76% and 98%. In another embodiment, the polymer is chitosan with a number average molecular weight ( Mₙ) between 1kDa to 10MDa or between 2.7 kDa and 298 kDa. In still another embodiment, the blood component: polymer ratio is 3:1 (v/v).

According to a second aspect, the present application provides a polymer composition comprising chitosan, hydrochloric acid, NaCl and a blood component and a polymer composition consisting essentially of chitosan, hydrochloric acid, NaCl and a blood component. In an embodiment, the polymer composition is prepared with a chitosan solution having a chitosan concentration of about 1.62 % w/w. In another embodiment, the polymer composition is prepared with a hydrochloric acid solution having a hydrochloric concentration of about 38 mM. In still another embodiment, the polymer composition is prepared with a NaCl solution having a NaCl concentration of about 160 mM. Various embodiments with respect to the polymer composition pH, osmolality have been presented and do apply herein. Various embodiments of the chitosan degree and molecular weight have been presented and do apply herein. Various embodiment of the blood component have been presented and do apply herein.

According to a third aspect, the present application provides a method for repairing a tissue in a subject in need thereof, said method comprising the step of introducing into said tissue a polymer composition as defined herein such that the polymer composition adheres to the tissue and promotes cell proliferation for repairing the tissue. In an embodiment, the tissue is selected from the group consisting of cartilage, meniscus, ligament, tendon, bone, skin, cornea, periodontal tissues, maxillofacial tissues, temporomandibular tissues, abscesses, resected tumors and ulcers.

According to a fourth aspect, the present application provides the use of a polymer composition as defined herein for repairing a tissue of a subject, wherein the polymer composition adheres to the tissue and promotes cell proliferation for repairing the tissue as well as the use of a polymer composition as defined herein in the manufacture of a medicament for repairing a tissue of a subject, wherein the polymer composition adheres to the tissue and promotes cell proliferation. In an embodiment, the tissue is selected from the group consisting of cartilage, meniscus, ligament, tendon, bone, skin, cornea, periodontal tissues, maxillofacial tissues, temporomandibular tissues, abscesses, resected tumors and ulcers.

According to a fifth aspect, the present application provides a polymer composition as defined herein for repairing a tissue in a subject, wherein the polymer composition adheres to the tissue and promotes cell proliferation. In an embodiment, the tissue is selected from the group consisting of cartilage, meniscus, ligament, tendon, bone, skin, cornea, periodontal tissues, maxillofacial tissues, temporomandibular tissues, abscesses, resected tumors and ulcers.

According to a sixth aspect, the present application provides a method of preparing a polymer composition for repairing a tissue in a subject. Broadly, the method comprises (or consists essentially of) the step of: dissolving chitosan in HCl to provide a chitosan-HCl mixture; adding a NaCl solution to the chitosan-HCl mixture to provide a chitosan-HCl-NaCl mixture; and admixing at least one blood component to the chitosan-HCl-NaCl mixture to provide the polymer composition. In an embodiment, the chitosan is dissolved in HCl by heating at a temperature of 60°C. In a further embodiment, the concentration of chitosan in the chitosan-HCl-NaCl mixture is about 1.62 % w/w of chitosan, the concentration of hydrochloric acid in the chitosan-HCl-NaCl mixture is about 38 mM of hydrochloric acid and/or the concentration of NaCl in the chitosan-HCl-NaCl mixture is about 160 mM Various embodiments of the blood component, the pH of the polymer composition the osmolalityof the polymer composition, the polymer, the chitosan and the inorganic salt have been described and do apply herein.

In a particular embodiment, the polymer composition comprises 1.62 % w/w of chitosan, 38 mM of hydrochloric acid and 160 mM of NaCl mixed with blood.

It is also provided herein a method for repairing a tissue of a patient, the method comprising the step of introducing into the tissue a polymer gel composition as defined herein such that the composition adheres to the tissue and promotes cell recruitment and other events for repairing the tissue. The composition can be placed or injected into a body site where the mixture aids the tissue repair, regeneration and reconstruction.

In an embodiment, the tissue is selected from the group consisting of cartilage, meniscus, ligament, tendon, bone, skin, cornea, periodontal tissues, maxillofacial tissues, temporomandibular tissues, abscesses, resected tumors, and ulcers.

It is also provided herein a method of preparing a polymer composition for repairing tissues in a subject, the method comprising the steps of dissolving chitosan in HCl; adding a NaCl solution to the chitosan-HCl mixture; and admixing at least one blood component to the chitosan-HCl-NaCl.

In an additional embodiment, the chitosan is dissolved in HCl by heating at 60°C for about 2 hours.

It is also provided herein the use of the composition as defined herein for repairing a tissue of a patient and/or in the manufacture of a medicament for repairing a tissue of a patient, wherein the composition adhere to the tissue and promote cell proliferation for repairing the tissue.

### BRIEF DESCRIPTION OF THE DRAWINGS

Reference will now be made to the accompanying drawings.
Fig. 1 is a histogram of the coagulation time of blood/chitosan clots, prepared with chitosan number average molecular weight (*M*ₙ) of 75 kDa; 79% DDA, showing that all the blood/chitosan mixtures coagulated within 15 minutes and that blood/chitosan-HCl-NaCl mixtures coagulated faster than blood/chitosan-HCl-βGP (β Glycerol Phophate) mixtures in 3 of 4 rabbits.
Fig. 2 is a histogram of the mechanical strength of blood/chitosan-HCl-NaCl and blood/chitosan-HCl-βGP clots prepared with chitosan *M*ₙ 75 kDa; 79% DDA with blood from four different rabbits.
Fig. 3 is a histogram of the coagulation time of blood/chitosan clots, prepared with chitosan *M*ₙ 2.7 kDa; 98% DDA, showing that all the blood/chitosan mixtures coagulated within 18 minutes and that blood/chitosan-HCl-NaCl mixtures clotted faster than blood/chitosan-HCl-βGP mixtures.
Fig. 4 is a histogram of the mechanical strength of blood/chitosan-HCl-NaCl and blood/chitosan-HCl-βGP clots prepared with chitosan *M*ₙ 2.7 kDa; 98% DDA in triplicate.
Fig. 5 is a histogram of the coagulation time of blood/chitosan clots, prepared with chitosan *M*ₙ 75 kDa; 79% DDA, showing that all the blood/chitosan mixtures coagulated within 13 minutes and that blood/chitosan-HCl-NaCl mixtures clotted faster than blood/chitosan-HCl-βGP mixtures.
Fig. 6 is a histogram of the mechanical strength of blood/chitosan-HCl-NaCl and blood/chitosan-HCl-βGP clots prepared with chitosan *M*ₙ 75 kDa; 79% DDA in triplicate.
Fig. 7 is a histogram of the coagulation time of blood/chitosan clots, prepared with chitosan *M*ₙ 232 kDa; 81% DDA, showing that all the blood/chitosan-HCl-NaCl and blood/chitosan-HCl-βGP mixtures coagulated within 7 minutes.
Fig. 8 is a histogram of the mechanical strength of blood/chitosan-HCl-NaCl and blood/chitosan-HCl-βGP clots prepared with chitosan *M*ₙ 232 kDa; 81%DDA in triplicate.
Fig. 9 is a histogram of the coagulation time of blood/chitosan clots, prepared with chitosan *M*ₙ 298 kDa; 76% DDA, showing that all the blood/chitosan-HCl-NaCl and blood/chitosan-HCl-βGP mixtures coagulated within 7 minutes.
Fig. 10 is a histogram of the mechanical strength of blood/chitosan-HCl-NaCl and blood/chitosan-HCl-βGP clots prepared with chitosan *M*ₙ 298 kDa; 76% DDA in triplicate.

### DETAILED DESCRIPTION

It is provided a novel polymer composition comprising a blood component, a polymer and at least one inorganic salt. Related uses and methods are also provided. In an embodiment, such composition can be used for repairing a tissue in a subject. In yet a further embodiment, such composition can be used to limit the time associated with the coagulation of blood in a chitosan composition.

The polymer composition comprises a polymer, such as, for example chitosan, chitin, hyaluronan, glycosaminoglycan, chondroitin sulfate, keratin sulfate, dermatan sulfate, heparin and heparin sulfate. The polymer should be able to form a gel and be used for the treatment of tissue repair.

The polymer composition also comprises a blood component. Any blood component is contemplated herein, such as whole blood, processed blood, venous blood, arterial blood, blood from bone, blood from bone-marrow, bone marrow, umbilical cord blood or placenta blood. In an embodiment, the blood component is derived from whole blood to be enriched or depleted for a specific blood component. In a preferred embodiment, the mix ratio between the blood component and the polymer is about 3:1

The polymer composition also comprises at least one inorganic salt. In a preferred embodiment, NaCl is used. Alternatively, any inorganic salt, including sodium, chloride, potassium, calcium, magnesium, phosphate, sulfate, carboxylate salt, such as KCl, CsCl, CaCl₂, CsF, KClO₄, NaNO₃ and CaSO₄ are also contemplated herein.

The polymer composition can further comprise an acid, such as a mineral acid or an organic acid. The acid is used to lower the pH of the composition to facilitate dissolution of the chitosan. In a preferred embodiment, HCl is used as the acid. Alternatively, acetic acid, nitric acid, phosphoric acid, sulfuric acid, boric acid, hydrofluoric acid or hydrobromic acid could also be used.

In another embodiment, the polymer composition consists essentially of a blood component, a polymer and at least one inorganic salt. Such polymer composition does not contain additional components which participates to the formation of the gel or coagulation of the blood (such as coagulation products for example) but can contain other components such as an acid (to facilitate the dissolution of the chitosan), preservatives, etc.

It is specifically described herein the formulation of chitosan and a blood component, under physiological conditions (pH, osmolality). Briefly chitosan-HCl-NaCl solutions are mixed with blood, to create blood/chitosan-HCl-NaCl. The blood/chitosan-HCl-NaCl compositions are capable of forming a gel. In addition, the blood component in the blood/chitosan-HCl-NaCl is capable of coagulating to form an implant for tissue repair. In addition, when chitosan having a number average molecular weight lower than *M*ₙ 232 kDa is used, the chitosan-HCl-NaCl solutions mixed with blood described herein coagulates faster than chitosan-HCl-βGP solutions.

A preferred embodiment is shown in Example 1 where a novel formulation of physiological chitosan-HCl-NaCl solution was prepared with chitosan *M*ₙ 75 kDa; 79% DDA and mixed with whole blood. Blood/chitosan-HCl-NaCl mixtures coagulated faster than blood/chitosan-HCl-βGP mixtures to create fast-coagulating blood/chitosan implants for tissue repair.

Chitosan (1.62%w/w)-HCl (38 mM)-NaCl (160 mM) solution at pH of 6.50 and osmolality of 354 mOsm/kg, and chitosan (1.62%w/w)-HCl (71 mM)-βGP (2.15%w/w) solution at pH of 6.65 and osmolality of 334 mOsm/kg (Table 1) were mixed with fresh rabbit whole blood. To prepare chitosan/blood clots, immediately following collection of whole blood, a volume of 900 µl of whole blood was mixed into a cryotube containing 300 µl of 1.62% chitosan-HCl-NaCl solution (or 1.62% chitosan-HCl-PGP solution) and three 0.39 g stainless steel balls. The mixture was shaken by hand for 10 seconds (the mix ratio of blood to chitosan was 3 to 1).

250 µl was transferred into 3 glass tubes at 37°C with a 1 ml syringe to prepare 3 clots (implants). One clot was used to test coagulation time and fixed immediately after it coagulated, the second clot was used to test coagulation time and fixed at 60 minutes after mixing, and the third clot was used to test coagulation time and mechanical strength after 60 minutes.

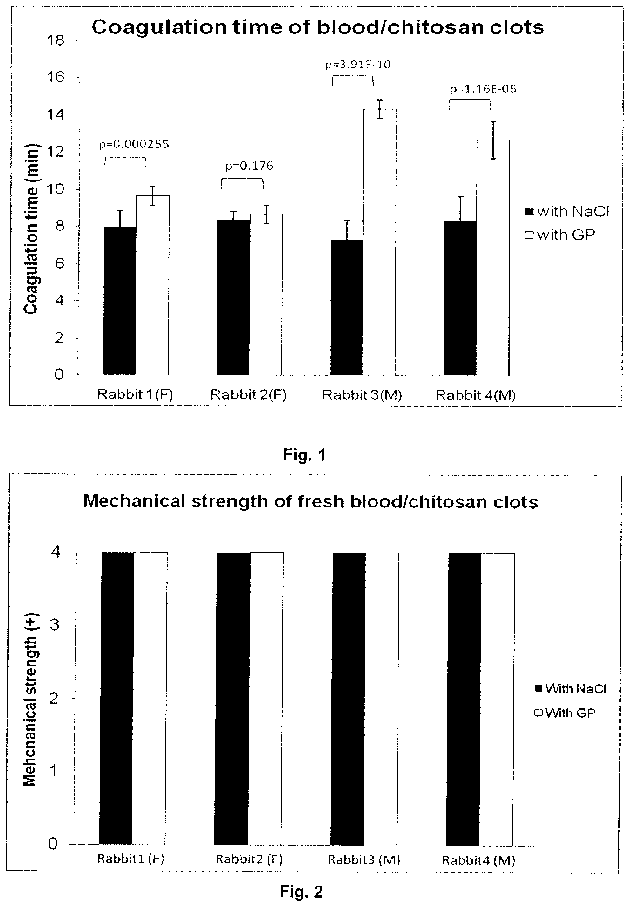

Coagulation time results (Tables 2-5 and Fig. 1) showed that all blood/chitosan mixtures prepared with chitosan *M*ₙ 75 kDa; 79% DDA coagulated within 15 minutes (from 6 minutes to 15 minutes). In 3 of 4 rabbits, the coagulation time of the blood/chitosan-HCl-NaCl mixtures was less than the coagulation time of the blood/chitosan-HCl-βGP mixtures (p < 0.05). In the best case, coagulation time was shortened by a factor of 2. The mechanical strength results (Table 6 and Fig. 2) demonstrated that all the fresh blood/chitosan mixture clots were firm and elastic gel, the mechanical strength of all the clots was scored as "++++".

After 60 minutes, some serum exuded from the clots (Table 6). The homogeneity evaluation results are shown in Table 7.

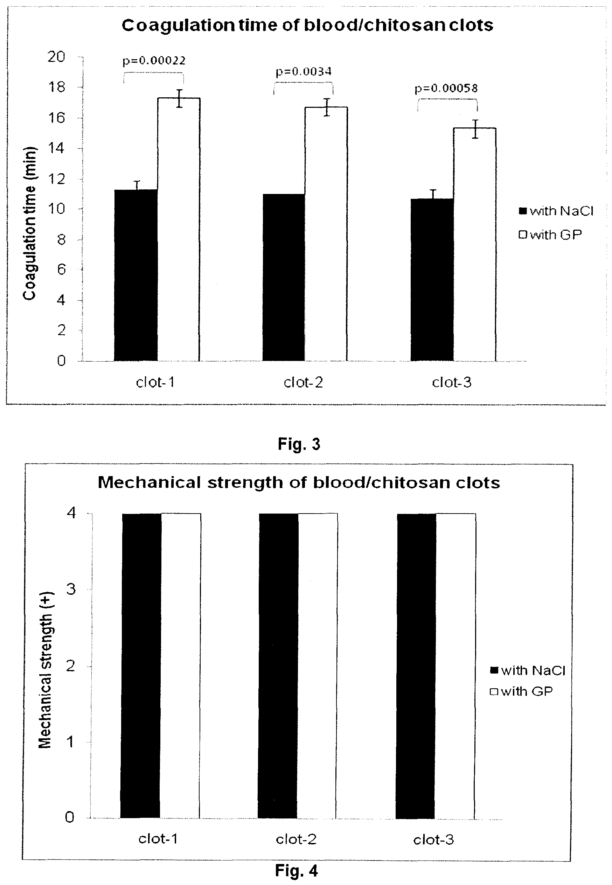

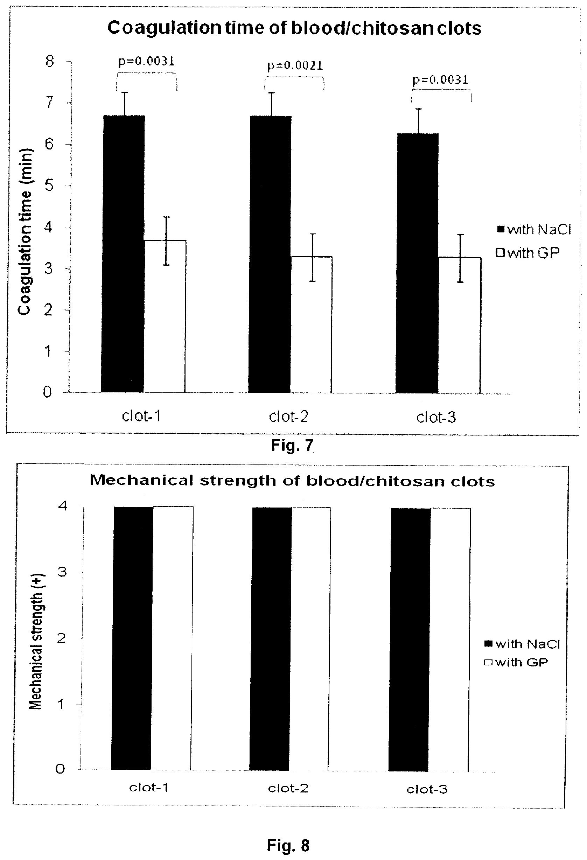

Another embodiment is shown in Example 2 where chitosans of different molecular weights *M*ₙ were used to prepare a novel formulation of physiological chitosan-HCl-NaCl mixtures (Table 8). Blood/chitosan-HCl-NaCl mixtures coagulated faster than blood/chitosan-HCl-βGP mixtures when the chitosan has a number average molecular weight *M*ₙ <232 kDa to create fast-coagulating blood/chitosan-HCl-NaCl implants with good mechanical strength for tissue repair (Tables 9-12 and Figs. 3-6). Chitosans with number average molecular weights *M*ₙ >232 kDa were also used to prepare a novel formulation of physiological chitosan-HCl-NaCl mixtures that coagulated within 7 minutes to create fast-coagulating blood/chitosan implants with good mechanical strength (Tables 13-16 and Figs. 7-10).

After 60 minutes, some serum exuded from the clots and the homogeneity evaluation results are shown in Table 17.

It is thus demonstrated herein that blood/chitosan-HCl-NaCl mixtures coagulated faster than blood/chitosan-βGP mixtures when the chitosan had a molecular weight *M*ₙ < 232 kDa to create blood/chitosan clots (implants) with good mechanical properties. Chitosans with a number average molecular weight *M*ₙ > 232 kDa were also used to prepare blood/chitosan-HCl-NaCl mixtures that coagulated to create blood/chitosan clots (implants) with good mechanical properties. Therefore, these solutions can be alternatives to the current chitosan-HCl-βGP solution known in the art with the advantage of solidifying faster.

The compositions described herein can be used to improve the repair and to regenerate cartilaginous tissues and other tissues including without limitation meniscus, ligament, tendon, bone, skin, cornea, periodontal tissues, abscesses, resected tumors, and ulcers.

There is also contemplated herein the use of the polymer compositions described herein that can be placed or injected into a body site where the mixture aids the repair, regeneration, reconstruction or bulking of tissues. Repaired tissues include for example without limitation cartilage, meniscus, ligament, tendon, bone, skin, cornea, periodontal tissues, abscesses, resected tumors, and ulcers. The tissue that can be repaired or regenerated is for example without limitation cartilage, meniscus, ligament, tendon, bone, skin, cornea, periodontal tissue, abscesses, resected tumors, or ulcers. In some cases, the site of introduction in the body may be surgically prepared to remove abnormal tissues. Such procedure can be done by piercing, abrading or drilling into adjacent tissue regions or vascularized regions to create channels for the polymer composition to migrate into the site requiring repair.

The present disclosure will be more readily understood by referring to the following examples which are given to illustrate embodiments rather than to limit its scope.

### EXAMPLE 1

### Formulation and characterization of fresh blood/physiological chitosan solution mixtures (Rabbit blood)

### 1-Preparation of Chitosan (1.62% w/w)-HCl (38 mM)-NaCl (160 mM) solution; without NaOH, pH: 6.6; total volume: 10.0 ml

0.180 g of chitosan (degree of deacetylation or DDA = 79% and number average weight or *M*ₙ 75kDa) was weighted in a 20 ml beaker. H₂Odd was added to the beaker until the weight of chitosan + H₂O = 9.34 g. A magnetic stir bar was added into the beaker and the solution was stirred for about 10 minutes in order to hydrate the chitosan powder as much as possible. 0.38 ml of HCl 1 N (Sigma, Product N° 318949) was added to the solution under moderate stirring. The beaker was covered with parafilm™, and the solution was heated to about 60°C for 2 hours, stirred overnight until completely dissolved. 0.32 ml of 5N NaCl (Sigma, Product N° S-9888) solution was added into the beaker and mixed. The obtained pH of the chitosan solution was physiological at 6.5 and the osmolality was also physiological at 354 mOsm/kg (Table 1).

### 2- Preparation of Chitosan (1.62% w/w)-HCl (71 mM)-βGP (2.15%) solution, pH: 6.6, total volume: 9.0 ml

0.162 g of chitosan (degree of deacetylation or DDA = 79% and number average weight or *M*ₙ = 75kDa) was weighted in a 20 ml beaker. H₂Odd was added to the beaker until the weight of chitosan + H₂O=6.65g. A magnetic stir bar was added into the beaker to stir the solution for about 10 minutes in order to hydrate the chitosan powder as much as possible. 0.55 ml of HCl 1 N (Sigma, Product N° 318949) was added to the solution under moderate stirring. The beaker was covered with parafilm™ and stirred overnight until completely dissolved. 1.8 ml of 10.75% βGP (Sigma, Product N° G9891) in 50 mM HCl solution was added into the beaker and mixed (mix ratio of chitosan solution/βGP solution is 4:1). The obtained pH of the chitosan solution was physiological at 6.7 and the osmolality was also physiological at 334 mOsm/kg (Table 1).

**Table 1**

| Composition and properties of chitosan solutions. | | | | | | | |
|---|---|---|---|---|---|---|---|
| **Solution No and type.** | **C_{chitosan} (%w/w)** | **C_{HCl} (mM)** | **C_{NaCl} (mM)** | **C_{βGP} (%w/w)** | **Precipitation** | **pH (Measured)** | **Osmolality (mOsm/kg)** |
| Chitosan-HCl-NaCl pH 6.6 | 1.62 | 38 | 160 | - | no | 6.50 | 354 |
| Chitosan-HCl-βGP pH 6.6 | 1.62 | 71 | - | 2.15 | no | 6.65 | 334 |

### 3-Drawing blood

Blood was extracted from rabbits using sterile technique, starting by injecting 0.3 cc/kg Hypnorm® IM to the rabbits (for example 0.9 cc for a 3 kg rabbit). First, for each rabbit, ∼ 2 ml of blood was collected in a Vacutainer® tube containing EDTA (Fisher, BD, Product N° 02-683-99A) to obtain CBC (complete blood count) and platelet count. Second, for each rabbit, ∼ 5mL of blood was collected using a sterile 5cc syringe (Fisher, BD, Product N° 309604). Four rabbits were used in this experiment.

### 4- Preparing blood/physiological chitosan solution mixtures, measuring the coagulation time and mechanical strength of the clots

To prepare blood/chitosan-HCl-NaCl (pH 6.6) clots-1, immediately following collection of whole blood, a 900 µl pipet of whole blood was added into a cryotube containing three 0.39 g stainless steel balls and 300 µl chitosan solution and mixed by hand shaking for 10 seconds. 250 µl was then transferred into 3 glass tubes at 37°C with a 1 ml syringe to prepare 3 clots: the first clot was used to test coagulation time and fixed immediately after it coagulated; the second clot was used to test coagulation time and fixed at 60 minutes after mixing; the third clot was used to test coagulation time and mechanical strength after 60 minutes. This experiment was accomplished in triplicate for each rabbit. To prepare blood/chitosan-HCl-βGP (pH 6.6) clots-2, the same procedure was repeated as described hereinabove, but a chitosan-HCl-βGP (pH 6.6) solution was mixed with blood.

Coagulation was determined by visualization of the clot at 37°C. All the three glass tubes were used for testing coagulation time. The glass tubes were gently taken from the hot plate vertically every minute, slowly tilted, and the blood mixture was visualized at the bottom of tube. If the mixture was immobile and formed clot, it was coagulated; if the mixture was still mobile at the bottom of the tube, it was not coagulated yet. Mechanical strength was tested by putting the clot on the centre of the palm and pressing the clot with a finger until it was crushed. The resistance to compression, liquid expression and crushed appearance were also observed. The mechanical strength was scored with a 4 "+" system: "+" represents clot was easily broken and crushed appearance was multiple fragments (more than 5 fragments); "++" represents clot was relatively firm and crushed appearance was multiple fragments (3-5 fragments); "+++" represents clot was firm and elastic, crushed appearance was 2-3 fragments; "++++" represents clot was firm and elastic, crushed appearance was 2 fragments (sometimes still connected) or there was just a hole in the center of clot.

For Rabbit 259F (R1F), all the blood/chitosan mixtures coagulated within 10 minutes (from 7 minutes to 10 minutes) and formed firm elastic clots (see Table 2). The coagulation time of blood/chitosan-HCl-NaCl pH 6.6 mixtures (8 minutes) was significantly less than the coagulation time of blood/chitosan-HCl-βGP pH 6.6 mixtures (9.7 minutes) (Fig. 1).

**Table 2**

| Coagulation time of blood/chitosan clots from rabbit R259F (R1F). | | | | |
|---|---|---|---|---|
| **Mixture** | **Sample number** | | **Coagulation time (Min)** | **Mean value (Min** |
| Fresh blood/chitosan-HCl-NaCl pH 6.6 (clot1) | Mixture 1 | 1 | 8 | 8 |
| | | 2 | 8 | |
| | | 3 | 8 | |
| | Mixture 2 | 1 | 9 | |
| | | 2 | 9 | |
| | | 3 | 9 | |
| | Mixture 3 | 1 | 7 | |
| | | 2 | 7 | |
| | | 3 | 7 | |
| Fresh blood/chitosan-HCl-βGP pH 6.6 (clot2) | Mixture 1 | 1 | 10 | 9.7 |
| | | 2 | 10 | |
| | | 3 | 10 | |
| | Mixture 2 | 1 | 9 | |
| | | 2 | 9 | |
| | | 3 | 9 | |
| | Mixture 3 | 1 | 10 | |
| | | 2 | 10 | |
| | | 3 | 10 | |

For Rabbit 260F (R2F), all the blood/chitosan mixtures coagulated within 9 minutes and formed firm elastic clots (see Table 3 and Fig. 1).

**Table 3**

| Coagulation time of blood/chitosan clots from rabbit R260F (R2F) | | | | |
|---|---|---|---|---|
| **Mixture** | **Sample number** | | **Coagulation time (Min)** | **Mean value (Min)** |
| Fresh blood/chitosan-HCl-NaCl pH 6.6 (clot1) | Mixture 1 | 1 | 9 | 8.3 |
| | | 2 | 9 | |
| | | 3 | 9 | |
| | Mixture 2 | 1 | 8 | |
| | | 2 | 8 | |
| | | 3 | 8 | |
| | Mixture 3 | 1 | 8 | |
| | | 2 | 8 | |
| | | 3 | 8 | |
| Fresh blood/chitosan-HCl-βGP pH 6.6 (clot2) | Mixture 1 | 1 | 9 | 8.7 |
| | | 2 | 9 | |
| | | 3 | 9 | |
| | Mixture 2 | 1 | 9 | |
| | | 2 | 9 | |
| | | 3 | 9 | |
| | Mixture 3 | 1 | 8 | |
| | | 2 | 8 | |
| | | 3 | 8 | |

For Rabbit 261M (R3M), all the blood/chitosan mixtures coagulated within 15 minutes (from 6 minutes to 15 minutes) and formed firm elastic clots (see Table 4). The coagulation time of blood/chitosan-HCl-NaCl pH 6.6 mixtures (7.3 minutes) was significantly less than the coagulation time of blood/chitosan-HCl-βGP pH 6.6 mixtures (14.3 minutes) (Fig. 1).

**Table 4**

| Coagulation time from rabbit R261M (R3M) | | | | |
|---|---|---|---|---|
| **Mixture** | **Sample number** | | **Coagulation time (Min)** | **Mean value (Min)** |
| Fresh blood/chitosan-HCl-NaCl pH 6.6 (clot1) | Mixture 1 | 1 | 6 | 7.3 |
| | | 2 | 6 | |
| | | 3 | 6 | |
| | Mixture 2 | 1 | 8 | |
| | | 2 | 8 | |
| | | 3 | 8 | |
| | Mixture 3 | 1 | 8 | |
| | | 2 | 8 | |
| | | 3 | 8 | |
| Fresh blood/chitosan-HCl-βGP pH 6.6 (clot2) | Mixture 1 | 1 | 14 | 14.3 |
| | | 2 | 14 | |
| | | 3 | 14 | |
| | Mixture 2 | 1 | 15 | |
| | | 2 | 15 | |
| | | 3 | 15 | |
| | Mixture 3 | 1 | 14 | |
| | | 2 | 14 | |
| | | 3 | 14 | |

For Rabbit 262M (R3MR4M), all the blood/chitosan mixtures coagulated within 14 minutes (from 7 minutes to 14 minutes) and formed firm elastic clots (see Table 5). The coagulation time of blood/chitosan-HCl-NaCl pH 6.6 mixtures (8.3 minutes) was significantly less than the coagulation time of blood/chitosan-HCl-βGP pH 6.6 mixtures (12.7 minutes) (Fig. 1).

**Table 5**

| Coagulation time from rabbit R262M (R4M) | | | | |
|---|---|---|---|---|
| **Mixture** | **Sample number** | | **Coagulation time (Min)** | **Mean value (Min)** |
| Fresh blood/chitosan-HCl-NaCl pH 6.6 (clot1) | Mixture 1 | 1 | 7 | 8.3 |
| | | 2 | 7 | |
| | | 3 | 7 | |
| | Mixture 2 | 1 | 8 | |
| | | 2 | 8 | |
| | | 3 | 8 | |
| | Mixture 3 | 1 | 10 | |
| | | 2 | 10 | |
| | | 3 | 10 | |
| Fresh blood/chitosan-HCl-βGP pH 6.6 (clot2) | Mixture 1 | 1 | 12 | 12.7 |
| | | 2 | 12 | |
| | | 3 | 12 | |
| | Mixture 2 | 1 | 12 | |
| | | 2 | 12 | |
| | | 3 | 12 | |
| | Mixture 3 | 1 | 14 | |
| | | 2 | 14 | |
| | | 3 | 14 | |

The mechanical strength results (Table 6 and Fig. 2) demonstrated that all the fresh blood/chitosan clots were firm and elastic, the mechanical strengths of all the clots was scored as "++++". After 60 minutes, there was some serum exuded from the clots (scored as ++ in most cases). Although the mechanical strength scores were identical for all clots, 9 of 12 βGP clots (clot2) had just a hole in the center while only 3 of 12 NaCl clots (clot1) had just a hole in the center, suggesting an improvement in mechanical strength.

**Table 6**

| Mechanical strength test of blood/chitosan clots. | | | | | |
|---|---|---|---|---|---|
| **Rabbit No.** | **Mixture component** | **Sample No.** | **Resistance to compression** | **Liquid expressed** | **Crushed appearance** |
| **R259F** | Fresh blood/chitosan-HCl-NaCl pH 6.6 (clot1) | Clot1-1 | Firm and elastic ++++ | Some liquid exuded ++ | Break into 2 fragments but still connected |
| | | Clot1-2 | Firm and elastic ++++ | Some liquid exuded ++ | Break into 2 fragments but still connected |
| | | Clot1-3 | Firm and elastic ++++ | Some liquid exuded ++ | Break into 2 fragments but still connected |
| | Fresh blood/chitosan-HCl-βGP pH 6.6 (clot2) | Clot2-1 | Firm and elastic ++++ | Some liquid exuded ++ | Not broken but a hole in the center of clot |
| | | Clot2-2 | Firm and elastic ++++ | Some liquid exuded ++ | Not broken but a hole in the center of clot |
| | | Clot2-3 | Firm and elastic ++++ | Some liquid exuded ++ | Break into 2 fragments but still connected |
| **R260F** | Fresh blood/chitosan-HCl-NaCl pH6.6 (clot1) | Cfot1-1 | Firm and elastic ++++ | Some liquid exuded ++ | Break into 2 fragments but still connected |
| | | Clot1-2 | Firm and elastic ++++ | Some liquid exuded ++ | Break into 2 fragments but still connected |
| | | Clot1-3 | Firm and elastic ++++ | Some liquid exuded ++ | Break into 2 fragments but still connected |
| | Fresh blood/chitosan-HCl-βGP pH 6.6 (clot2) | Clot2-1 | Firm and elastic ++++ | Some liquid exuded ++ | Not broken but a hole in the center of clot |
| | | Clot2-2 | Firm and elastic ++++ | Some liquid exuded ++ | Not broken but a hole in the center of clot |
| | | Clot2-3 | Firm and elastic ++++ | Some liquid exuded ++ | Not broken but a hole in the center of clot |
| **R261M** | Fresh blood/chitosan-HCl-NaCl pH 6.6 (clot1) | Clot1-1 | Firm and elastic ++++ | Some liquid exuded ++ | Not broken but a hole in the center of clot |
| | | Clot1-2 | Firm and elastic ++++ | Some liquid exuded ++ | Not broken but a hole in the center of clot |
| | | Clot1-3 | Firm and elastic ++++ | Some liquid exuded ++ | Break into 2 fragments but still connected |
| | Fresh blood/chitosan-HCl-βGP pH 6.6 (clot2) | Clot2-1 | Firm and elastic ++++ | Some liquid exuded ++ | Not broken but a hole in the center of clot |
| | | Clot2-2 | Firm and elastic ++++ | Some liquid exuded ++ | Not broken but a hole in the center of clot |
| | | Clot2-3 | Firm and elastic ++++ | Some liquid exuded ++ | Not broken but a hole in the center of clot |
| **R262M** | Fresh blood/chitosan-HCl-NaCl pH 6.6 (clot1) | Clot1-1 | Firm and elastic ++++ | Some liquid exuded ++ | Break into 2 fragments but still connected |
| | | Clot1-2 | Firm and elastic ++++ | Some liquid exuded +++ | Not broken but a hole in the center of clot |
| | | Clot1-3 | Firm and elastic ++++ | Some liquid exuded + | Break into 2 fragments but still connected |
| | Fresh blood/chitosan-HCl-βGP pH 6.6 (clot2) | Clot2-1 | Firm and elastic ++++ | Some liquid exuded ++ | Not broken but a hole in the center of clot |
| | | Clot2-2 | Firm and elastic ++++ | Some liquid exuded ++ | Not broken but a hole in the center of clot |
| | | Clot2-3 | Firm and elastic ++++ | Some liquid exuded ++ | Break into 2 fragments but still connected |

### 5- Histological treatment and homogeneity evaluation of blood/chitosan clots

Fixed clots were sectioned in two parts. One part was cryoprotected with sucrose, infiltrated with OCT, cryosectioned, stained with Safranin O/Fast Green and observed by optical microscopy. One part was stored at 4°C until further use. All the photos were taken from the blood/chitosan clot samples fixed 60 minutes after the clots were prepared; two photos taken with 5x and 40x objectives from different regions of each sample were used for homogeneity evaluation. Each specimen was observed under microscopy with special emphasis on: presence of bubbles or cracks; presence and distribution of precipitates of chitosan described as: large aggregates or small aggregates; chitosan distribution and whether or not they are homogeneously dispersed across the section; erythrocyte morphology in terms of discoid, shrunken, swollen or chaining.

Histology showed that the homogeneity of clots prepared with βGP was better (8 of 12 samples scored as "+" and none scored as "-") than the homogeneity of the clots prepared with NaCl (3 of 11 samples scored as "+" and 3 of 11 samples scored as "-") (Table 7).

**Table 7**

| Homogeneity of blood/chitosan clots. | | | |
|---|---|---|---|
| **Clot-1 sample (with NaCl)** | **Homogeneity of clot-1** | **Clot-2 sample (with βGP)** | **Homogeneity of clot-2** |
| FB 1-1-R259F | + | FB 2-1-R259F | + |
| FB 1-2-R259F | + | FB 2-2-R259F | + |
| FB 1-3-R259F | - | FB 2-3-R259F | ± |
| FB 1-1-R260F | + | FB 2-1-R260F | + |
| FB 1-2-R260F | ± | FB 2-2-R260F | + |
| FB 1-3-R260F | - | FB 2-3-R260F | + |
| FB 1-1-R261M | ± | FB 2-1-R261M | ± |
| FB 1-2-R261M | - | FB 2-2-R261M | + |
| FB 1-3-R261M | * | FB 2-3-R261M | + |
| FB 1-1-R262M | ± | FB 2-1-R262M | + |
| FB 1-2-R262M | ± | FB 2-2-R262M | ± |
| FB 1-3-R262M | + | FB 2-3-R262M | ± |

| | | | |
|---|---|---|---|
| * data not available | | | |

### EXAMPLE 2

### Formulation and characterization of fresh blood/chitosan clots by using chitosan solutions with different molecular weight (Rabbit blood)

### 1-Preparation of Chitosan (1.62% w/w)-HCl (38 mM)-NaCl (160 mM) solution; without NaOH, pH: 6.6; total volume: 10.0 ml

The chitosan-HCl-NaCl solutions were prepared as described previously with chitosans of different *M*ₙ and DDA (lot No. AS-144-02-A: *M*ₙ 2.7 kDa and DDA 98%; lot No. CH10075: *M*ₙ 75 kDa and DDA 79%; lot No. CH0100702B: *M*ₙ 232 kDa, and DDA 81%; lot No. CH0050602A: *M*ₙ 298 kDa andDDA 76%). The pH of the chitosan solutions were physiological (6.2 to 6.7) and the osmolality was also physiological (326 to 356 mOsm/kg) (see Table 8).

### 2- Preparation of Chitosan (1.62% w/w)-HCl (71 mM)-βGP (2.15%) solution, pH: 6.6, total volume: 9.0 ml

The chitosan-HCl-NaCl solutions were prepared as described previously with chitosans of different *M*ₙ and DDA (lot No. AS-144-02-A: *M*ₙ 2.7 kDa and DDA 98%; lot No. CH10075: *M*ₙ 75 kDa and DDA 79%; lot No. CH0100702B: *M*ₙ 232 kDa, and DDA 81%; lot No. CH0050602A: *M*ₙ 298 kDa and DDA 76%). The pH of the chitosan solutions were physiological at 6.7 and the osmolality was also physiological (340 to 345 mOsm/kg) (Table 8).

**Table 8**

| Composition and properties of chitosan solutions | | | | | | | |
|---|---|---|---|---|---|---|---|
| **Solution No and type.** | **C_{chitosan} (%w/w)** | **C_{HCl} (mM)** | **C_{NaCl} (mM)** | **C_{βGP} (%w/w)** | **Precipitation** | **pH (Measured)** | **Osmolarity (mOsm)** |
| Chitosan-HCl-NaCl pH6.6(AS-144-02-A) | 1.62 | 55 | 160 | no | no | 6.19 | 356 |
| Chitosan-HCl-βGP pH6.6(AS-144-02-A) | 1.62 | 77 | No | 2.15 | no | 6.69 | 345 |
| Chitosan-HCl-NaCl pH6.6(CH10075) | 1.62 | 38 | 160 | no | no | 6.62 | 326 |
| Chitosan-HCl-βGP pH6.6(CH10075) | 1.62 | 71 | No | 2.15 | no | 6.67 | 344 |
| Chitosan-HCl-NaCl pH6.6(CH0100702B) | 1.62 | 38 | 160 | no | no | 6.65 | 339 |
| Chitosan-HCl-βGP pH6.6(CH01007028) | 1.62 | 71 | No | 2.15 | no | 6.71 | 337 |
| Chitosan-HCl-NaCl pH6.6(CH0050602A) | 1.62 | 38 | 160 | no | no | 6.58 | 332 |
| Chitosan-HCl-βGP pH6.6(CH0050602A) | 1.62 | 71 | No | 2.15 | no | 6.68 | 340 |

### 3- Drawing blood

Blood was extracted from rabbits using sterile technique, as described previously. Four rabbits were used in this experiment.

### 4- Preparing blood/physiological chitosan solution mixtures, measuring the coagulation time and mechanical strength of the clots

Rabbit whole blood/chitosan-HCl-NaCl mixtures and rabbit whole blood/chitosan-HCl-βGP mixtures were prepared at a mix ratio of 3:1 v/v as described previously. This experiment was accomplished in triplicate for each rabbit. Coagulation of the clot was determined by visualization of the clot at 37°C as described previously. Mechanical strength was tested as described previously.

For chitosan lot No. AS-144-02-A (*M*ₙ of 2.7 kDa and 98% DDA), all the blood/chitosan mixtures coagulated within 18 minutes (from 10 minutes to 18 minutes) and formed firm elastic clots (see Table 9). The coagulation time of blood/chitosan-HCl-NaCl pH 6.6 mixtures was significantly less than the coagulation time of blood/chitosan-HCl-βGP pH 6.6 mixtures (Fig. 3). The mechanical strength test results showed that all the clots were firm and elastic, the mechanical strengths of all the clots was scored as "++++" and the clots did not retract significantly (see Table 10 and Fig. 4).

**Table 9**

| Coagulation time of fresh blood/chitosan mixtures(Lot No. AS-144-02-A) | | | | | |
|---|---|---|---|---|---|
| **Mixture** | **Concentration of chitosan solution and mixing ratio** | | **Coagulation time (Min)** | **Mean value (Min)** | **Comments** |
| Fresh blood/ Chitosan-HCl-NaCl | 1.62% and 3:1 (clot1-1-1) | 1 | 12 | 11.3 | All the mixture samples coagulated within 12 minutes (from 10 minutes to 12 minutes). |
| | | 2 | 11 | | |
| | | 3 | 11 | | |
| | 1.62% and 3:1 (clot1-1-2) | 1 | 11 | 11 | |
| | | 2 | 11 | | |
| | | 3 | 11 | | |
| | 1.62% and 3:1 (clot1-1-3) | 1 | 11 | 10.7 | |
| | | 2 | 10 | | |
| | | 3 | 11 | | |
| Fresh blood/ Chitosan-HCl-βGP | 1.62% and 3:1 (clot1-2-1) | 1 | 18 | 17.3 | All the mixture samples coagulated within 18 minutes (from 15 minutes to 18 minutes). |
| | | 2 | 17 | | |
| | | 3 | 17 | | |
| | 1.62% and 3:1 (clot1-2-2) | 1 | 17 | 16.7 | |
| | | 2 | 16 | | |
| | | 3 | 17 | | |
| | 1.62% and 3:1 (clot1-2-3) | 1 | 15 | 15.3 | |
| | | 2 | 15 | | |
| | | 3 | 16 | | |

**Table 10**

| Mechanical strength test of fresh blood/chitosan clots ( Lot No. AS-144-02-A) | | | |
|---|---|---|---|
| **Sample** | **Resistance to compression** | **Liquid expressed** | **Crushed appearance** |
| Fresh blood/Chitosan-HCl-NaCl clot1-1-1 (1.62%, 3:1) | Firm and elastic ++++ | Almost no liquid expressed + | 2 fragments |
| Fresh blood/Chitosan-HCl-NaCl clot1-1-2 (1.62%, 3:1) | Firm and elastic ++++ | Almost no liquid expressed + | 2 fragments |
| Fresh blood/Chitosan-HCl-NaCl clot1-1-3 (1.62%, 3:1) | Firm and elastic ++++ | A few liquid expressed ++ | 2 fragments |
| Fresh blood/Chitosan-HCl-βGP clot1-2-1 (1.62%, 3:1) | Firm and elastic ++++ | Almost no liquid expressed + | 2 fragments |
| Fresh blood/Chitosan-HCl-βGP clot1-2-2 (1.62%, 3:1) | Firm and elastic ++++ | Almost no liquid expressed + | 2 fragments but still connected |
| Fresh blood/Chitosan-HCl-βGP clot1-2-3 (1.62%, 3:1) | Firm and elastic ++++ | Almost no liquid expressed + | 2 fragments |

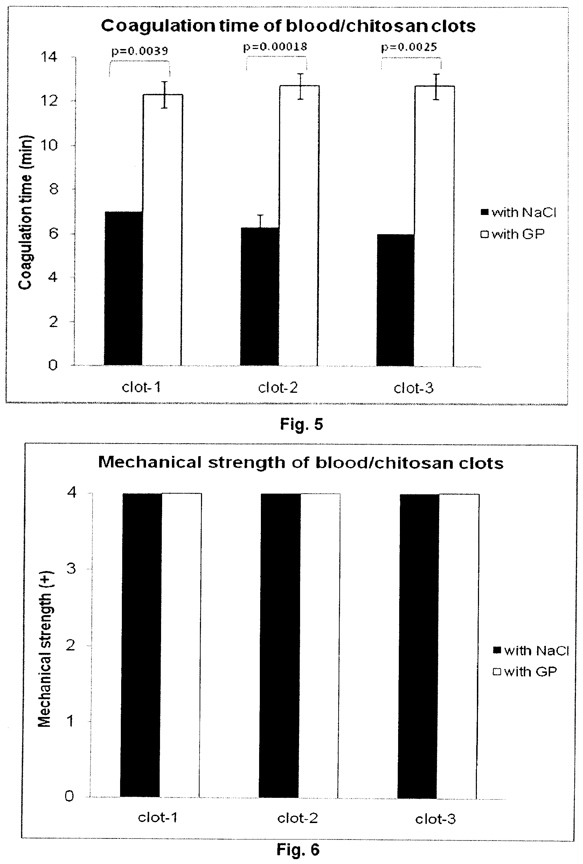

For chitosan lot No. CH10075 (*M*ₙ of 75 kDa and 79% DDA), all the blood/chitosan mixtures coagulated within 13 minutes (from 6 minutes to 13 minutes) and formed firm elastic clots (Table 11). The coagulation time of blood/chitosan-HCl-NaCl pH 6.6 mixtures was significantly less than the coagulation time of blood/chitosan-HCl-βGP pH 6.6 mixtures (Fig. 5). The mechanical strength test results showed that all the clots were firm and elastic, the mechanical strengths of all the clots was scored as "++++" and the clots did not retract significantly (see Table 12 and Fig. 6).

**Table 11**

| Coagulation time of fresh blood/chitosan mixtures (Lot No. CH10075) | | | | | |
|---|---|---|---|---|---|
| **Mixture** | **Concentration of chitosan solution and mixing ratio** | | **Coagulation time (Min)** | **Mean value (Min)** | **Comments** |
| Fresh blood/ Chitosan-HCl-NaCl | 1.62% and 3:1 (clot2-1-1) | 1 | 7 | 7 | All the mixture samples coagulated within 7 minutes (from 6 minutes to 7 minutes). |
| | | 2 | 7 | | |
| | | 3 | 7 | | |
| | 1.62% and 3:1 (clot2-1-2) | 1 | 6 | 6.3 | |
| | | 2 | 7 | | |
| | | 3 | 6 | | |
| | 1.62% and 3:1 (clot2-1-3) | 1 | 6 | 6 | |
| | | 2 | 6 | | |
| | | 3 | 6 | | |
| Fresh blood/ Chitosan-HCl-βGP | 1.62% and 3:1 (clot2-2-1) | 1 | 12 | 12.3 | All the mixture samples coagulated within 13 minutes (from 12 minutes to 13 minutes). |
| | | 2 | 12 | | |
| | | 3 | 13 | | |
| | 1.62% and 3:1 (clot2-2-2) | 1 | 13 | 12.7 | |
| | | 2 | 13 | | |
| | | 3 | 12 | | |
| | 1.62% and 3:1 (clot2-2-3) | 1 | 13 | 12.7 | |
| | | 2 | 12 | | |
| | | 3 | 13 | | |

**Table 12**

| Mechanical test of fresh blood/chitosan clots (Lot No. CH10075) | | | |
|---|---|---|---|
| **Sample** | **Resistance to compression** | **Liquid expressed** | **Crushed appearance** |
| Fresh blood/Chitosan-HCl-NaCl clot2-1-1 (1.62%, 3:1) | Firm and elastic ++++ | Almost no liquid expressed + | 2 fragments but still connected |
| Fresh blood/Chitosan-HCl-NaCl clot2-1-2 (1.62%, 3:1) | Firm and elastic ++++ | Almost no liquid expressed + | 2 fragments |
| Fresh blood/Chitosan-HCl-NaCl clot2-1-3 (1.62%, 3:1) | Firm and elastic ++++ | Almost no liquid expressed + | 2 fragments |
| Fresh blood/Chitosan-HCl-βGP clot2-2-1 (1.62%, 3:1) | Firm and elastic ++++ | Almost no liquid expressed + | 2 fragments but still connected |
| Fresh blood/Chitosan-HCl-βGP clot2-2-2 (1.62%, 3:1) | Firm and elastic ++++ | Almost no liquid expressed + | 2 fragments |
| Fresh blood/Chitosan-HCl-βGP clot2-2-3 (1.62%, 3:1) | Firm and elastic ++++ | Almost no liquid expressed + | 2 fragments but still connected |

For chitosan lot No. CH0100702B (*M*ₙ of 232 kDa and 81% DDA), all the blood/chitosan mixtures coagulated within 7 minutes (from 3 minutes to 7 minutes) and formed firm elastic clots (see Table 13 and Fig. 7). The mechanical strength test results showed that all the clots were firm and elastic, the mechanical strengths of all the clots was scored as "++++"and the clots did not retract significantly (see Table 14 and Fig. 8).

**Table 13**

| Coagulation time of fresh blood/chitosan mixture (Lot No. CH0100702B) | | | | | |
|---|---|---|---|---|---|
| **Mixture** | **Concentration of chitosan solution and mixing ratio** | | **Coagulation time (Min)** | **Mean value (Min)** | **Comments** |
| Fresh blood/ Chitosan-HCl-NaCl | 1.62% and 3:1 (clot3-1-1) | 1 | 7 | 6.7 | All the mixture samples coagulated within 7 minutes (from 6 minutes to 7 minutes). |
| | | 2 | 7 | | |
| | | 3 | 6 | | |
| | 1.62% and 3:1 (clot3-1-2) | 1 | 7 | 6.7 | |
| | | 2 | 7 | | |
| | | 3 | 6 | | |
| | 1.62% and 3:1 (clot3-1-3) | 1 | 7 | 6.3 | |
| | | 2 | 6 | | |
| | | 3 | 6 | | |
| Fresh blood/ Chitosan-HCl-βGP | 1.62% and 3:1 (clot3-2-1) | 1 | 3 | 3.7 | All the mixture samples coagulated within 4 minutes (from 3 minutes to 4 minutes). |
| | | 2 | 4 | | |
| | | 3 | 4 | | |
| | 1.62% and 3:1 (clot3-2-2) | 1 | 4 | 3.3 | |
| | | 2 | 3 | | |
| | | 3 | 3 | | |
| | 1.62% and 3:1 (clot3-2-3) | 1 | 3 | 3.3 | |
| | | 2 | 3 | | |
| | | 3 | 4 | | |

**Table 14**

| Mechanical test of fresh blood/chitosan clots (Lot No. CH0100702B) | | | |
|---|---|---|---|
| **Sample** | **Resistance to compression** | **Liquid expressed** | **Crushed appearance** |
| Fresh blood/Chitosan-HCl-NaCl clot3-1-1 (1.62%, 3:1) | Firm and elastic ++++ | Almost no liquid expressed + | 2 fragments |
| Fresh blood/Chitosan-HCl-NaCl clot3-1-2 (1.62%, 3:1) | Firm and elastic ++++ | Almost no liquid expressed ++ | 2 fragments |
| Fresh blood/Chitosan-HCl-NaCl clot3-1-3 (1.62%, 3:1) | Firm and elastic ++++ | Almost no liquid expressed + | 2 fragments but still connected |
| Fresh blood/Chitosan-HCl-βGP clot3-2-1 (1.62%, 3:1) | Firm and elastic ++++ | Almost no liquid expressed ++ | 2 fragments but still connected |
| Fresh blood/Chitosan-HCl-βGP clot3-2-2 (1.62%, 3:1) | Firm and elastic ++++ | Almost no liquid expressed + | 2 fragments but still connected |
| Fresh blood/Chitosan-HCl-βGP clot3-2-3 (1.62%, 3:1) | Firm and elastic ++++ | Almost no liquid expressed + | 2 fragments but still connected |

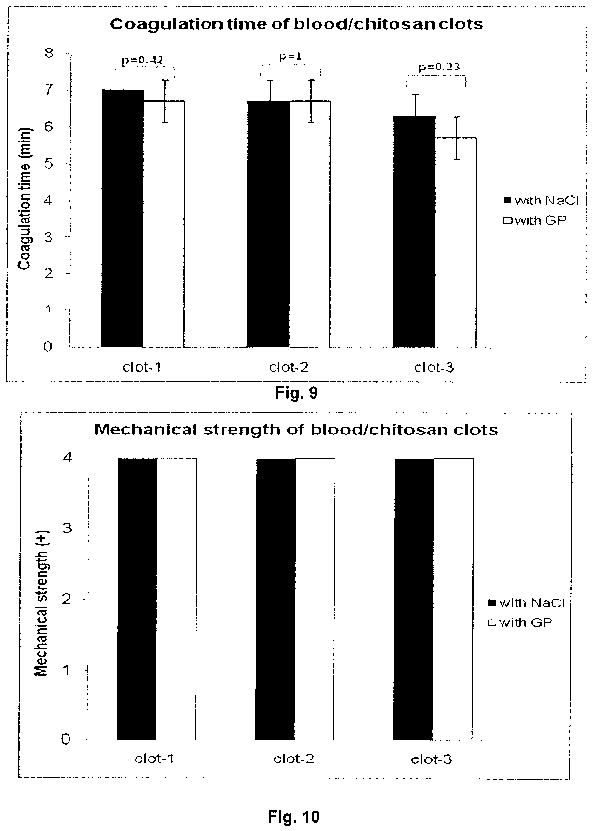

For chitosan lot No. CH0050602A (*M*ₙ of 298 kDa, 76% DDA), all the blood/chitosan mixtures coagulated within 7 minutes (from 5 minutes to 7 minutes) and formed firm elastic clots (see Table 15 and Fig. 9). The mechanical strength test results showed that all the clots were firm and elastic, the mechanical strengths of all the clots was scored as "++++" and the clots did not retract significantly (see Table 16 and Fig. 10).

**Table 15**

| Coagulation time of fresh blood/chitosan mixture (Lot No. CH0050602A) | | | | | |
|---|---|---|---|---|---|
| **Mixture** | **Concentration of chitosan solution and mixing ratio** | | **Coagulation time (Min)** | **Mean value (Min)** | **Comments** |
| Fresh blood/ Chitosan-HCl-NaCl | 1.62% and 3:1 (clot4-1-1) | 1 | 7 | 7 | All the mixture samples coagulated within 7 minutes (from 6 minutes to 7 minutes). |
| | | 2 | 7 | | |
| | | 3 | 7 | | |
| | 1.62% and 3:1 (clot4-1-2) | 1 | 7 | 6.7 | |
| | | 2 | 7 | | |
| | | 3 | 6 | | |
| | 1.62% and 3:1 (clot4-1-3) | 1 | 7 | 6.3 | |
| | | 2 | 6 | | |
| | | 3 | 6 | | |
| Fresh blood/ Chitosan-HCl-βGP | 1.62% and 3:1 (clot4-2-1) | 1 | 7 | 6.7 | All the mixture samples coagulated within 7 minutes (from 5 minutes to 7 minutes). |
| | | 2 | 6 | | |
| | | 3 | 7 | | |
| | 1.62% and 3:1 (clot4-2-2) | 1 | 7 | 6.7 | |
| | | 2 | 7 | | |
| | | 3 | 6 | | |
| | 1.62% and 3:1 (clot4-2-3) | 1 | 6 | 5.7 | |
| | | 2 | 6 | | |
| | | 3 | 5 | | |

**Table 16**

| Mechanical test of fresh blood/chitosan clots (Lot No. CH0050602A) | | | |
|---|---|---|---|
| **Sample** | **Resistance to compression** | **Liquid expressed** | **Crushed appearance** |
| Fresh blood/Chitosan-HCl-NaCl clot4-1-1 (1.62%, 3:1) | Firm and elastic ++++ | Almost no liquid expressed + | 2 fragments |
| Fresh blood/Chitosan-HCl-NaCl clot4-1-2 (1.62%, 3:1) | Firm and elastic ++++ | Almost no liquid expressed + | 2 fragments but still connected |
| Fresh blood/Chitosan-HCl-NaCl clot4-1-3 (1.62%, 3:1) | Firm and elastic ++++ | Almost no liquid expressed + | 2 fragments but still connected |
| Fresh blood/Chitosan-HCl-βGP clot4-2-1 (1.62%, 3:1) | Firm and elastic ++++ | Almost no liquid expressed + | Hole in center |
| Fresh blood/Chitosan-HCl-βGP clot4-2-2 (1.62%, 3:1) | Firm and elastic ++++ | Almost no liquid expressed + | 2 fragments but still connected |
| Fresh blood/Chitosan-HCl-βGP clot4-2-3 (1.62%, 3:1) | Firm and elastic ++++ | Almost no liquid expressed + | 2 fragments but still connected |

### 5- Histological treatment and homogeneity evaluation of blood/chitosan clots

Histological treatment and homogeneity evaluation was performed as described hereinabove.

Histology showed that the homogeneity of clots prepared with βGP was better (9 of 12 samples scored as "+" and none scored as "-") than the homogeneity of the clots prepared with NaCl (2 of 12 samples scored as "+" and 7 of 12 samples scored as "-") (see Table 17).

**Table 17**

| Homogeneity of blood/chitosan clots | | | |
|---|---|---|---|
| **Clot samples (with NaCl)** | **Homogeneity of clots (with NaCl)** | **Clot samples (with βGP)** | **Homogeneity of clots (with βGP)** |
| blood 1-1-1 (AS-144-02-A) | + (between ± and +) | blood 1-2-1 (AS-144-02-A) | ± |
| blood 1-1-2 (AS-144-02-A) | ± (between ± and +) | blood 1-2-2 (AS-144-02-A) | + (between ± and +) |
| blood 1-1-3 (AS-144-02-A) | ± (between ± and +) | blood 1-2-3 (AS-144-02-A) | + (between ± and +) |
| blood 2-1-1 (CH10075) | - | blood 2-2-1 (CH10075) | + |
| blood 2-1-2 (CH10075) | - | blood 2-2-2 (CH10075) | + (between ± and +) |
| blood 2-1-3 (CH10075) | - | blood 2-2-3 (CH10075) | + |
| blood 3-1-1 (CH0100702B) | + | blood 3-2-1 (CH0100702B) | + |
| blood 3-1-2 (CH0100702B) | + | blood 3-2-2 (CH0100702B) | + |
| blood 3-1-3 (CH0100702B) | - | blood 3-2-3 (CH0100702B) | + |
| blood 4-1-1 (CH0050602A) | - | blood 4-2-1 (CH0050602A) | + |
| blood 4-1-2 (CH0050602A) | - | blood 4-2-2 (CH0050602A) | + |
| blood 4-1-3 (CH0050602A) | - | blood 4-2-3 (CH0050602A) | + |

The invention is as defined in the appended claims. While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth, and as follows in the scope of the appended claims.

## Claims

1. A blood/chitosan-HCl-NaCl composition comprising:
a) whole blood; and
b) a chitosan solution comprising: chitosan having an average molecular weight of Mₙ < 232kDa, HCl, and NaCl at a concentration of 160mM;
wherein the blood/chitosan-HCl-NaCl composition has a pH between 6.0 and 7.8; and
wherein the blood/chitosan-HCl-NaCl composition coagulates faster than a blood/chitosan-HCl-β-glycerol phosphate composition.

2. The blood/chitosan-HCl-NaCl composition of claim 1, wherein the polymer composition has a pH between 6.2 and 6.7, and more preferably a pH of 6.6.

3. The blood/chitosan-HCl-NaCl composition of claim 1 or 2, wherein chitosan degree of deacetylation (DDA) is between 20% to 100%, and more preferably between 76% and 98%.

4. The blood/chitosan-HCl-NaCl composition of any one of claims 1 to 3, wherein the whole blood: chitosan ratio is 3:1 v/v.

5. The blood/chitosan-HCl-NaCl composition of Claim 1, wherein the HCI is at a concentration of 38 mM.

6. A blood/chitosan-HCl-NaCl composition as defined in any one of claims 1 to 5, for use in repairing a tissue in a subject, wherein the polymer composition adheres to the tissue and promotes cell proliferation.

7. The blood/chitosan-HCl-NaCl composition for use according to claim 6, wherein said tissue is selected from the group consisting of cartilage, meniscus, ligament, tendon, bone, skin, cornea, periodontal tissues, maxillofacial tissues, temporomandibular tissues, abscesses, resected tumors and ulcers.

## Patentansprüche

1. Blut/Chitosan-HCl-NaCl-Zusammensetzung, umfassend:
a) Vollblut; und
b) eine Chitosan-Lösung, umfassend: Chitosan mit einem mittleren Molekulargewicht von Mₙ < 232 kDa, HCl, und NaCl in einer Konzentration von 160 mM;
wobei die Blut/Chitosan-HCl-NaCl-Zusammensetzung einen pH zwischen 6,0 und 7,8 aufweist; und
wobei die Blut/Chitosan-HCl-NaCl-Zusammensetzung schneller koaguliert als eine Blut/Chitosan-HCl-β-Glycerolphosphat-Zusammensetzung.

2. Blut/Chitosan-HCl-NaCl-Zusammensetzung nach Anspruch 1, wobei die Polymer-Zusammensetzung einen pH zwischen 6,2 und 6,7 und bevorzugter einen pH von 6,6 aufweist.

3. Blut/Chitosan-HCl-NaCl-Zusammensetzung nach Anspruch 1 oder 2, wobei der Deacetylierungsgrad (DDA) von Chitosan zwischen 20 % und 100 % und bevorzugter zwischen 76 % und 98 % liegt.

4. Blut/Chitosan-HCl-NaCl-Zusammensetzung nach einem der Ansprüche 1 bis 3, wobei das Verhältnis von Vollblut:Chitosan 3:1 (v/v) beträgt.

5. Blut/Chitosan-HCl-NaCl-Zusammensetzung nach Anspruch 1, wobei die HCl in einer Konzentration von 38 mM vorliegt.

6. Blut/Chitosan-HCl-NaCl-Zusammensetzung wie nach einem der Ansprüche 1 bis 5 definiert, zur Verwendung bei der Reparatur eines Gewebes bei einem Patienten, wobei die Polymer-Zusammensetzung an dem Gewebe haftet und die Zellproliferation fördert.

7. Blut/Chitosan-HCl-NaCl-Zusammensetzung zur Verwendung nach Anspruch 6, wobei das genannte Gewebe aus der Gruppe ausgewählt ist, bestehend aus Knorpel, Menisken, Ligamenten, Sehnen, Knochen, Haut, Korneae, periodontalen Geweben, maxillofazialen Geweben, mandibulotemporalen Geweben, Abszessen, resezierten Tumoren und Ulzera.

## Revendications

1. Composition de sang/chitosane-HCl-NaCl comprenant :
a) du sang entier ; et
b) une solution de chitosane comprenant : du chitosane ayant une masse moléculaire moyenne Mₙ < 232 kDa, du HCl et du NaCl à une concentration de 160 mM ;
la composition de sang/chitosane-HCl-NaCl ayant un pH de 6,0 à 7,8 ;
et
la composition de sang/chitosane-HCl-NaCl coagulant plus rapidement qu'une composition de sang/chitosane-HCl-phosphate de β-glycérol.

2. Composition de sang/chitosane-HCl-NaCl selon la revendication 1, la composition polymère ayant un pH de 6,2 à 6,7, et plus préférablement un pH de 6,6.

3. Composition de sang/chitosane-HCl-NaCl selon la revendication 1 ou 2, dans laquelle le degré de désacétylation (DDA) du chitosane est de 20 % à 100 %, et plus préférablement de 76 % à 98 %.

4. Composition de sang/chitosane-HCl-NaCl selon l'une quelconque des revendications 1 à 3, dans laquelle le rapport sang entier:chitosane est de 3:1 v/v.

5. Composition de sang/chitosane-HCl-NaCl selon la revendication 1, dans laquelle le HCl est présent à une concentration de 38 mM.

6. Composition de sang/chitosane-HCl-NaCl telle que définie dans l'une quelconque des revendications 1 à 5, destinée à une utilisation dans la réparation d'un tissu chez un sujet, la composition polymère adhérant au tissu et favorisant la prolifération cellulaire.

7. Composition de sang/chitosane-HCl-NaCl destinée à une utilisation selon la revendication 6, ledit tissu étant sélectionné dans le groupe constitué des cartilages, des ménisques, des ligaments, des tendons, des os, de la peau, de la cornée, des tissus parodontaux, des tissus maxillo-faciaux, des tissus temporomandibulaires, des abcès, des tumeurs résectées et des ulcères.
